# EUROPEAN PATENT APPLICATION

(11) **EP 3 354 204 A1**
(43) Date of publication of application: **01.08.2018**
(21) Application number: 16848659.5
(22) Date of filing: 23.09.2016
(51) Int. Cl.: A61B 10/00, A61B 5/1455, G01N 21/17

(54) **BIOPHOTONIC MEASUREMENT DEVICE, INFORMATION PROCESSING PROGRAM, AND INFORMATION PROCESSING METHOD**

(30) Priority: 25.09.2015 JP 2015188713
(71) Applicant: NeU Corporation, Tokyo 101-0048 (JP)
(72) Inventor: HASEGAWA, Kiyoshi, Tokyo 105-8717 (JP); FUNANE, Tsukasa, Hiki-gun Saitama 350-0321 (JP)
(74) Representative: Strehl Schübel-Hopf & Partner
(86) International application number: PCT/JP2016/078019
(87) International publication number: WO 2017/051871

(57) **Abstract**

A biophotonic measurement device characterized by having: one or a plurality of photoirradiation means; one or a plurality of light detecting means; a mounting means for mounting the photoirradiation means and the light detecting means to a subject; an analysis means for calculating a state of mounting of the photoirradiation means and the light detecting means to the subject on the basis of a detection value measured by the light detecting means; and an output means for outputting the state of mounting using light or a voice, the output means being installed on the mounting means mounted to the subject, the photoirradiation means, or the light detecting means.

## Description

### TECHNICAL FIELD

The present invention pertains to a biophotonic measurement apparatus, an information processing program, and an information processing method.

### BACKGROUND ART

There has hitherto been provided an information processing system to acquire items of information representing activity states of a brain by: attaching, to a head region, a biophotonic measurement apparatus called a headset, including a plurality of probes and provided with a near infrared ray irradiation unit and a near infrared ray detection unit; detecting variations in bloodflow rate on a surface of the brain; and processing detected data by a data processing apparatus.

### [Documents of Prior Arts]

### [Patent Documents]

[Patent Document 1] Japanese Patent Application Laid-Open Publication No. 2005-13464
[Patent Document 2] Japanese Patent Application Laid-Open Publication No. 2012-161375

### SUMMARY OF THE INVENTION

### [Problems to be solved by the Invention]

A quality of measurement of the biophotonic measurement device depended on an attaching state of a probe in some cases. It is difficult to confirm the attaching state of the probe attached to a head region through a visual observation and other equivalent observations from outside. Stabilization of the quality of measurement requires attaining a higher efficiency of attaching the probe before the measurement. Such being the case, notification of the attaching state of the probe is required when attaching the biophotonic measurement apparatus.

The present invention aims at providing a biophotonic measurement apparatus that outputs an attaching state of the apparatus to a head region.

### [Means for solving the Problems]

Means given below are adopted for solving the problems described above.

To be specific, a first aspect is a biophotonic measurement apparatus including: one or a plurality of photo irradiation means to irradiate rays of light; one or a plurality of photodetection means to detect the rays of light of the photoirradiation means; attaching means to attach the photo irradiation means and the photodetection means to a measurement examinee; analyzing means to calculate an attaching state of the photoirradiation means and the photodetection means to the measurement examinee, based on a detection value detected by the photodetection means; and output means, installed on the attaching means or on the photoirradiation means or on the photodetection means attached to the measurement examinee, to output the attaching state by use of the rays of light or sounds/voices.

An aspect of the disclosure may be actualized such that an information processing apparatus runs a program. In other words, a configuration of the disclosure may be specified as a program run by an information processing apparatus that causes the respective means to execute processes in the aspect described above, or as a non-transitory computer readable recording medium on which this program is recorded. The configuration of the disclosure may also be specified as a method carried out by the information processing apparatus that causes the respective means to execute the processes. The configuration of the disclosure may further be specified as a system including the information processing apparatus that causes the respective means to execute the processes.

Steps describing the program contain, as a matter of course, the processes to be executed in time-series along a written sequence, and also processes that are not necessarily executed in time-series but executed in parallel or individually. Part of the steps describing the program may also be omitted.

### [Effect of the Invention]

According to the present invention, it is feasible to provide the biophotonic measurement apparatus that notifies the attaching state of the apparatus to the head region.

### BRIEF DESCRIPTION OF THE DRAWINGS

FIG. 1 is a diagram illustrating a configuration concerning information processing of an information processing system according to one embodiment of the present invention.
FIG. 2 is a diagram illustrating an example of a configuration of a head region attaching device.
FIG. 3 is a diagram illustrating an example of a configuration of a user terminal.
FIG. 4 is a view illustrating an example of such a state that the head region attaching device is attached to a head region of a user.
FIG. 5 is a view illustrating another example of the configuration of the head region attaching device.
FIG. 6 is a flowchart illustrating an example of an operation flow of an information processing system.

### DESCRIPTION OF THE EMBODIMENTS

An embodiment will hereinafter be described with reference to the drawings. A configuration of the embodiment is an exemplification, and a configuration of the invention is not limited to a specific configuration of the embodiment. When carrying out the invention, specific configurations corresponding to embodiments may also be properly adopted.

### [Embodiment]

### (Example of Configuration)

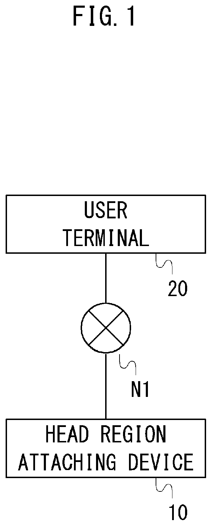

FIG. 1 is a diagram illustrating a configuration concerning information processing of the information processing system according to one embodiment of the present invention. As in FIG. 1, the information processing system includes a head region attaching device 10 and a user terminal 20. The information processing system detects measurement data (referred to also as detection values) representing the variations in bloodflow rate from the head region of a user, and thus acquires brain activity information representing the activity states of the user's brain. The information processing system is one example of the biophotonic measurement apparatus. The user is one example of a measurement examinee.

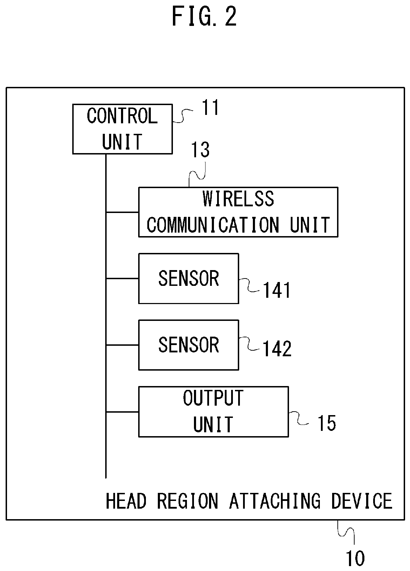

FIG. 2 is a diagram illustrating an example of a configuration of the head region attaching device. The head region attaching device 10 includes, in terms of an aspect of the information processing, a control unit 11, a wireless communication unit 13, sensors 141, 142, and an output unit 15. The sensors 141, 142 are generically termed sensors 14 when not distinguished therebetween. The control unit 11 controls measurements made by and communications with the head region attaching device 10. The control unit 11, which includes a processor instanced by a Central Processing unit (CPU) or a Data Signal Processor (DSP), and a memory, executes processing based on a computer program, firmware and other equivalent software deployed in an executable manner on the memory. However, the control unit 11 may also be a dedicated hardware circuit, a Field Programmable Gate Array (FPGA) and other equivalent circuits configured to execute cooperative processes with respective components by starting up the wireless communication unit 13, the sensors 141, 142 and the output unit 15. The control unit 11 may further be a mixed entity of the CPU, the DSP, the dedicated hardware circuit and other equivalent circuits . The head region attaching device 10 includes the two sensors 141, 142 herein and may also include three or more sensors 14.

The wireless communication unit 13 is connected via a predetermined interface to the control unit 11, the sensors 141, 142 and the output unit 15. However, the wireless communication unit 13 may also be configured to acquire the data from the sensors 141, 142 via the control unit 11. The wireless communication unit 13 performs communications with the user terminal 20 via a networkN1. The networkN1 is a network pursuant to Standards instanced by Bluetooth (registered trademark), wireless Local Area Network (LAN) and ZigBee. The wireless communication unit 13 is one example of transfer means. The information processing system is not, however, limited to such Standards of the wireless interface for the wireless communication unit 13.

There are two head region attaching devices 10-1, 10-2 (unillustrated), in which case, when performing the communications via the network N1, identifiers for identifying the head region attaching devices 10-1, 10-2 are embedded in a header field of a communication header, or in a user's data field (payload field) in communication data, thereby enabling the user terminal 20 to identify the user (measurement examinee) .

The information processing system may further be provided with a communication unit that performs wired communications in place of the wireless communication unit 13 or together with the wireless communication unit 13. In other words, the head region attaching device 10 and the user terminal 20 may also be connected together via an interface for the wired communications. In this case, it does not mean that there are limitations to the interface for the wired communications, but a variety of interfaces instanced by Universal Serial Bus (USB) and Peripheral Component Interconnect Express (PCI Express) are usable corresponding to applications of the information processing system.

The sensors 14 irradiate the head region with near infrared rays, then receive the near infrared rays partly absorbed and scattered in the vicinity of a brain cortex of the brain, and convert the received rays into electric signals. The brain cortex of the brain has a bloodflow rate that differs corresponding to, e.g., the activity states of the brain. As a result, in respective parts of the brain cortex of the brain, there occur variations in quantity of haemoglobin bound to oxygen in blood and a quantity of haemoglobin not bounded to the oxygen. An absorption characteristic or a scattering characteristic of the near infrared rays varies in the vicinity of the brain cortex of the brain due to the variations in haemoglobin quantity, variations in oxygen quantity and other equivalent variations. The sensors 14 convert the near infrared rays, into the electric signals, of which a light quantity varies based on variations in absorption rate or variations in transmittance of the near infrared rays, corresponding to such a state of the bloodflow in the vicinity of the brain cortex of the brain, and output the thus-converted electric signals. The respective sensors 14 are identified by, e.g., the identifiers.

The sensor 14 includes, e.g., a near infrared ray light source to irradiate the near infrared rays, and a light receiving unit to receive the near infrared rays. The near infrared ray light source is exemplified by a Light Emitting Diode (LED) and an infrared ray lamp. The light receiving unit includes: a photoelectric element instanced by a photo diode and a photo transistor; an amplifier; and an Analog-to-Digital (AD) converter. Note that the near infrared ray light source and the light receiving unit may not be paired when provided. For example, a plurality of light receiving units may be provided for one near infrared ray light source. The light receiving unit is one example of photodetection means. The sensor 14 may further include a light source for detecting an attachment state . The light source for detecting the attachment state is instanced by the LED. When the sensor 14 does not include the light source for detecting the attachment state, the near infrared ray light source substitutes for the light source for detecting the attachment state. Each of the light sources is an example of photoirradiation means.

The output unit 15 is exemplified by a light emitting element instanced by the LED, or by a loudspeaker to output sounds or voices and other equivalent devices, and displays output information given from the control unit 11. The output unit 15 outputs, as the light or the sounds/voices, the states of the sensors 141, 142 being attached to the head. The output unit 15 may also be a vibrator to output vibrations. The output unit 15 may further be provided in each sensor 14.

The user terminal 20 acquires, from the head region attaching device 10, variation data of the absorption rate or the transmittance of the near infrared rays in the vicinity of the brain cortex of the user's brain, and provides services including a variety of information processes related to the activity states of the user's brain. The user terminal 20 is one example of an information processing apparatus (computer) . The user terminal 20 may be attained by using a dedicated or general-purpose computer instanced by a Personal Computer (PC), a smartphone, a mobile phone, a tablet terminal, a car navigation system, a Personal Digital Assistant (PDA) and a game machine (amusement machine), or by using electronic equipment mounted with the computer. The user terminal 20 may be installed at, e.g., a fitness club, a cram school and other equivalent places.

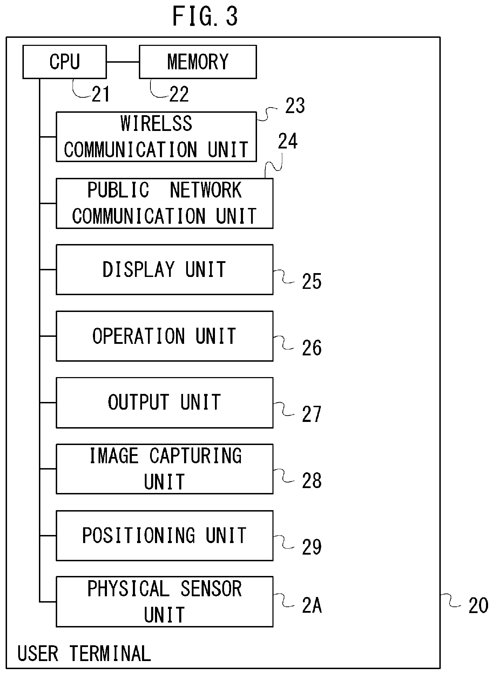

FIG. 3 is a diagram illustrating an example of a configuration of the user terminal. The user terminal 20 includes a CPU 21, a memory 22, a wireless communication unit 23, a public network communication unit 24, a display unit 25, an operation unit 26, an output unit 27, an image capturing unit 28, a positioning unit 29, and a physical sensor unit 2A. The CPU 21 executes processing as the user terminal 20, based on a computer program deployed in an executable manner on the memory 22. The processing as the user terminal 20 is, e.g., a service encompassing a variety of information processes related to the activity states of the user's brain. The CPU 21 running such a computer program is one example of analyzing means.

The memory 22 stores the computer program run by the CPU 21, or data processed by the CPU 21. The memory 22 may include a volatile memory and a nonvolatile memory.

The wireless communication unit 23 is the same as the wireless communication unit 13 of the head region attaching device 10. The wireless communication unit 23 is one example of receiving means. The user terminal 20 may also include a communication unit to perform the wired communications in place of the wireless communication unit 23 or together with the wireless communication unit 23.

The public network communication unit 24 performs communications (unillustrated) via a network N2 with a server, e.g., a server (arithmetic device) 3 and other equivalent devices on the network N2. The network N2 is a public network, and is exemplified by a mobile phone network. When the network N2 is the mobile phone network, the public network communication unit 24 establishes a connection to the network N2 via a base station of the mobile phone network. However, the network N2 may also be a network including: an access network to a communication apparatus of an Internet provider; and the Internet. The access network to the communication apparatus of the Internet provider is exemplified by an optical network provided by a common carrier, and Asymmetric Digital Subscriber Line (ADSL) . The network N2 is one example of a public wireless network. The public network communication unit 24 is one example of public wireless communication means . It does not, however, mean that the network N2 is limited to the public network in the information processing system; and the network N2 may also be an in-house network instanced by a Local Area Network (LAN), a private line of a business enterprise, an entrepreneur, a city hall, a school, a research institution and other equivalent organizations, and a wide area network instanced by a Virtual Private Network (VPN) . The business enterprise, the entrepreneur, the city hall, the school, the research institution and other equivalent organizations will hereinafter be simply referred to as the enterprise and other equivalent organizations.

The display unit 25, which is instanced by a liquid crystal display and an Electro-Luminescence (EL) panel, displays information outputted from the CPU 21. The operation unit 26, which is instanced by a push button and a touch panel, accepts user's operation. The output unit 27 is, e.g., a vibrator to output the vibrations, a loudspeaker to output the sounds or the voices, and other equivalent devices. The image capturing unit 28 is, e.g., a camera including a solid-state image capturing element. The solid-state image capturing element may involve making use of a Charge-Coupled Device (CCD) image sensor, a Complementary Metal Oxide Semiconductor (CMOS) image sensor, and other equivalent image sensors.

The positioning unit 29, which is instanced by Global Positioning System (GPS) receiver, receives radio waves from a GPS satellite, thereby calculating a present position (latitude, longitude, and other equivalent geographical coordinates), time and other equivalent data. It does not, however, mean that the positioning unit 29 is limited to a configuration including the GPS receiver. For example, when the public network communication unit 24 is applied to the mobile phone network, the positioning unit 29 may execute positioning based on a distance from the mobile phone base station.

The physical sensor unit 2A is, e.g., an acceleration sensor or an angular acceleration sensor, and other equivalent sensors. The physical sensor unit 2A may, however, be a temperature sensor, a humidity sensor, a barometric pressure sensor or a hydraulic pressure sensor.

### <Example of Head Region Attaching Device>

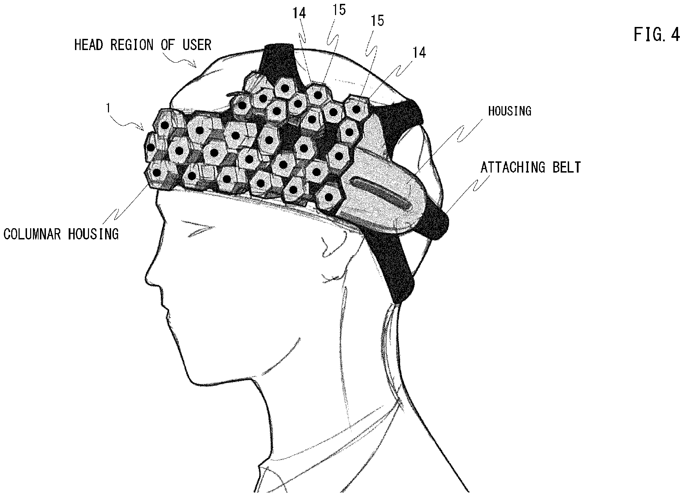

FIG. 4 is a view illustrating an example of such a state that the head region attaching device 10 is attached to the head region of the user. The head region attaching device 10 may take other configurations without being limited to the example of FIG. 4. In the example of FIG. 4, the head region attaching device 10 includes an attaching belt, a control box, the sensors 14 and the output units 15. The attaching belt is wound round in a headband shape and thus attached to the head region. The attaching belt, which is configured by an elastic body partly or entirely covering the head region, has a size smaller than the head region of the user at a normal time, but extends to a length suitable for the head region of the user and is tightly attached to the head region of the user at an attaching time. The attaching belt is fitted with a housing and a plurality of sensors 14. The housing includes: a control substrate for the control unit 11, the wireless communication unit 13 and other equivalent components; and a power source (battery) and other equivalent sources for actuating the head region attaching device 10. The output unit 15 is fitted to each sensor 14. Each sensor 14 and each output unit 15 are connected to the control box. The attaching belt has openings into which the sensors 14 are fitted, and the light source of the sensor 14 is mounted to enable irradiation of the light over the head region of the user. The light receiving unit of the sensor 14 is mounted to enable the irradiation of the light reflected from the head region of the user. The output unit 15, which is the light source instanced by the LED, is mounted to enable emitted light to be visually recognized from outside.

Each sensor 14 includes a housing taking a polygonal shape or a cylindrical shape in section. One section of the cylindrical housing is fitted with the sensor 14 including the light source or the light receiving unit, while the other section thereof is fitted with the output unit 15. The section may take, e.g., a hexagonal shape, an octagonal shape, a square shape, a circular shape and other equivalent shapes.

In the head region attaching device 10, holes to receive insertion of the columnar housings are arranged in the attaching belt in a way that takes a honeycomb shape (or a checkered pattern) with the same configuration as a sectional configuration of the columnar housings. The hole arrangement is not limited to the arrangement described above. The columnar housings are inserted into these holes, whereby each of the sensors 14 is arranged between the two neighboring sensors 14 on a line-by-line basis in a longitudinal direction of the attaching belt, thus taking such an appearance as to arrange the sensors 14 in a plurality of lines.

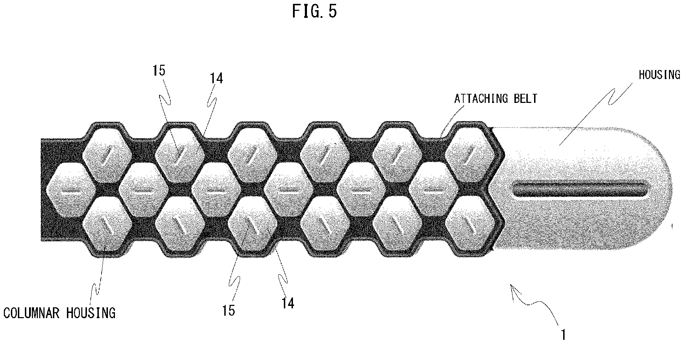

FIG. 5 is a view illustrating another example of the configuration of the head region attaching device 10. FIG. 5 depicts an example of the partial configuration of the head region attaching device 10. In the example of FIG. 5, the head region attaching device 10 takes the headband shape, and the attaching belt is fitted with the housing and the columnar housings including the sensors 14 and other equivalent components. The columnar housings are arranged in the honeycomb shape in the plurality of lines in the longitudinal direction of the attaching belt. The head region attaching device 10 in FIG. 5 has a structure of being
wound on, then attached to the head region of the user in the headband shape, and thus fixed to the head region of the user.

### (Operational Example)

In the information processing system, the CPU 21 of the user terminal 20 supports the user to align the sensors 14, based on an application program, for alignment (which will hereinafter be simply termed an alignment application), deployed in the executable manner on the memory 22. Such a process that the user terminal 20 supports the user to align the respective sensors 14, is also called calibration. Through the calibration, the user terminal 20 guides the user so that the respective sensors 14 are arranged in desirable positions of the head region of the user. The proper calibration is carried out, in which case it follows that the respective sensors 14 detect the variations in bloodflow rate in the desirable positions of the head region of the user.

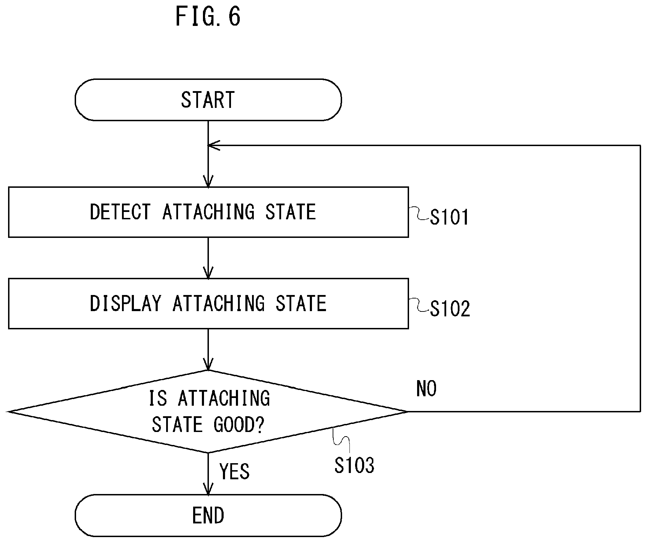

FIG. 6 is a flowchart illustrating an example of an operation flow of the information processing system. It is herein assumed that the head region attaching device 10 is attached to the head region of the user.

In S101, the user terminal 20 instructs the individual sensors 14 of the head region attaching device 10 to irradiate predetermined rays of light. The control unit 11 of the head region attaching device 10, upon receiving the instruction, causes the sensors 14 to irradiate the predetermined rays of light. The sensors 14 irradiate the predetermined rays of light from the light sources. When the head region attaching device 10 is attached to the head region of the user, the rays of light from the light sources of the sensors 14 are reflected by the head region and received by the light receiving units of the sensors 14. The sensors 14 convert the rays of light received by the light receiving units into the electric signals, and output these signals. The control unit 1 (11) of the head region attaching device 10 converts the electric signals outputted by the sensors 14 into measurement data (detection values). The detection value becomes larger as a quantity of the light received by the light receiving unit gets greater. The control unit 11 of the head region attaching device 10 transmits the detection values to the user terminal 20 via the wireless communication unit 23 by associating the detection values with the identifiers of the sensors 14. Herein, the detection values may be measured values themselves and may also be information into which the values measured for a fixed period are aggregated. The user terminal 20, when receiving the signals from the head region attaching device 10 via the wireless communication unit 23, stores the received signals in the memory 22.

The CPU 21 of the user terminal 20 determines whether the detection value of each sensor 14 is within a predetermined range (equal to or larger than a first predetermined value but less than a second predetermined value (first predetermined value < second predetermined value)), or less than the first predetermined value, or equal to or larger than the second predetermined value. When the detection value of the sensor 14 is within the predetermined range, this sensor 14 is considered to be properly attached to the head region. When the detection value of the sensor 14 is less than the first predetermined value, the quantity of the light reaching the light receiving unit of the sensor 14 is considered small. In other words, when the detection value of the sensor 14 is less than the first predetermined value, the sensor 14 is considered to be improperly attached such that the sensor 14 is positioned too far from the surface of the head region, or a large quantity of hairs are pinched between the sensor 14 and the head region (between the light source of the sensor 14 and the head region, or between the light receiving unit of the sensor 14 and the head region) . When the detection value of the sensor 14 is equal to or larger than the second predetermined value, the quantity of the light reaching the light receiving unit of the sensor 14 is considered large. In other words, when the detection value of the sensor 14 is equal to or larger than the second predetermined value, the sensor 14 is considered to be improperly attached such that the sensor 14 is positioned too near to the surface of the head region, or foreign matters (e.g., matters each having a high reflectance of the light) exist between the sensor 14 and the head region. The user terminal 20 stores a determination result per sensor 14 in the memory 22. Herein, a case of the detection value being less than the first predetermined value is defined as a first state, a case of the detection value being within the predetermined range (equal to or larger than the first predetermined value but less than the second predetermined value) is defined as a second state, and a case of the detection value being equal to or larger than the second predetermined value is defined as a third state.

In S102, the CPU 21 of the user terminal 20 transmits the determination result per sensor 14 to the head region attaching device 10 via the wireless communication unit 23. The head region attaching device 10, upon receiving the determination result per sensor 14 via the wireless communication unit 13, outputs the determination result to the output unit 15. For example, when the output unit 15 is provided as the light source per sensor 14, the determination result is displayed by the light. At this time, the output unit 15 displays, e.g., a yellow color in the case of the first state, a green color in the case of the second state, and a red color in the case of the third state. The second state corresponds to a good state, and hence the green color generally representing the goodness is displayed. The user wearing the head region attaching device 10 or an assistant for the user confirms the color displayed on the output unit 15, and is thereby enabled to confirm the attaching state of the head region attaching device 10. When the color displayed on the output unit 15 is the yellow or the red, the user wearing the head region attaching device 10 or the assistant for the user adjusts the position and other equivalent items of the sensor 14, and is thereby enabled to change the position of the sensor 14 to a proper position. It is feasible to easily recognize from the output unit 15 which sensor 14 is improperly attached.

In S103, the CPU 21 of the user terminal 20 determines whether the detection values of all of the sensors 14 are in the second state (good state). When the detection values of all of the sensors 14 are in the good state (S103; YES), the processing comes to an end. Whereas when the detection value of any one of the sensors 14 is not in the good state (S103; NO), the processing loops back to S101. The processing is iterated till the detection values of all of the sensors 14 reach the good state. This facilitates making an easy determination about the position of the sensor 14, which causes the improper attaching state of the head region attaching device 10, and therefore the attaching state of the sensor 14 may be easily adjusted. A further precise measurement may be made by preferably setting a mounting state of the sensor 14 when measuring the brain activity states.

The user terminal 20 determines herein whether the detection value of each of the sensors 14 of the head region attaching device 10 is in the good state, and the determination may, however, be made by the control unit 11 of the head region attaching device 10. At this time, the head region attaching device 10 may not transmit the detection value to the user terminal 20. The head region attaching device 10 is enabled to notify the user and other equivalent persons of the attaching state of the head region attaching device 10 without employing the user terminal 20.

### (Modified Example 1)

Though the exemplification described above has given the example of using the light source mounted in each sensor 14 as the output unit 15, some examples of notification of the attaching state through the sounds/voices will hereinafter be given. A modified example 1 has common points to the example described above, and hence the discussion will be focused on different points.

The output unit 15 of the head region attaching device 10 includes a loudspeaker. For example, the loudspeaker is mounted in the housing of FIG. 4. Identifying information instanced by a number is allocated to each of the sensors 14. The identifying information of the sensor 14 is written on each sensor 14 so as to be visually recognizable from outside.

The head region attaching device 10, when receiving the determination result per sensor 14 via the wireless communication unit 13, outputs the identifying information instanced by the number allocated to the sensor 14 with its detection value not being good by the sounds/voices from the loudspeaker as the output unit 15. The head region attaching device 10 is thereby enabled to notify the user and other equivalent persons of the sensor 14 in the improper attaching state.

### (Modified Example 2)

Though the exemplification described above has given the example of using the light source mounted in each sensor 14 as the output unit 15, an example of notifying the attaching state through vibrations will herein be described. A modified example 2 has common points to the example described above, and therefore the discussion will be focused on different points.

The output unit 15 of the head region attaching device 10 includes a vibrator to output the vibrations. For instance, the vibrator is mounted in the housing of FIG. 4. The head region attaching device 10, when receiving the determination result per sensor 14 via the wireless communication unit 13 and when all of the determination results are in the good state, outputs the vibrations in a predetermined pattern from the vibrator serving as the output unit 15. The head region attaching device 10 is thereby enabled to notify the user and other equivalent persons of the attaching state of the sensor 14. The user recognizes the vibrations and is thereby enabled to recognize the attaching state without visually recognizing the head region attaching device 10. The user wearing the head region attaching device 10 adjusts the attaching state of the head region attaching device 10 till recognizing the predetermined vibrations, and is thereby enabled to set the head region attaching device 10 in the good attaching state.

### (Modified Example 3)

Though the exemplification described above has given the example of using the light source mounted in each sensor 14 as the output unit 15, an example of notifying the attaching state by installing the light source in such a position as to enable the user wearing the head recognition attaching device 10 to make a visual recognition, will herein be described. A modified example 3 has common points to the example described above, and therefore the discussion will be focused on different points.

The output unit 15 of the head region attaching device 10 includes the light source mounted in such a position (corresponding to an upper part of an eye of the user) as to enable the user to make the visual recognition. The head region attaching device 10, when receiving the determination result per sensor 14 via the wireless communication unit 13 and when all of the determination results are in the good state, outputs greenish light from the light source as the output unit 15. The head region attaching device 10, when any one of the determination results is not in the good state, outputs reddish light from the light source as the output unit 15 . The head region attaching device 10 is thereby enabled to notify the user and other equivalent persons of the attaching state of the sensor 14. The user recognizes the light of the light source mounted in the position corresponding to the upper part of the eye of the user, and is thereby enabled to recognize the attaching state. The user wearing the head region attaching device 10 adjusts the attaching state of the head region attaching device 10 till the color of the light source becomes greenish, and is thereby enabled to set the head region attaching device 10 in the good attaching state.

### (Modified Example 4)

Though the exemplification described above has given the example that each sensor 14 includes the light source and the light receiving unit, there will herein be described such an example that each sensor 14 is separated into the light source and the light receiving unit, to which the light sources serving as the output units 15 are respectively fitted. A modified example 4 has common points to the example described above, and therefore the discussion will be focused on different points.

The head region attaching device 10, when receiving the determination result per sensor 14 via the wireless communication unit 13, outputs the determination result to each output unit 15. Hereat, for example, the output unit 15 displays the yellow color in the case of the first state, the green color in the case of the second state, and the red color in the case of the third state. The user wearing the head region attaching device 10 or the assistant for the user confirms the color displayed on the output unit 15, and is thereby enabled to confirm the attaching state of the head region attaching device 10. When a number of the sensors 14 not being good in state is smaller or larger than a predetermined value, the sensors 14 not being good in state may be rendered conspicuous by changing a lighting method (e.g., flickering) of the color to be displayed.

### (Modified Example 5)

Though the exemplification described above has given the example that the attaching state is displayed on the output unit 15, an example of displaying information other than the attaching state on the output unit 15, will herein be described. Amodified example 5 has common points to the example described above, and hence the discussion will be focused on different points.

Herein, the head region attaching device 10 outputs the colors corresponding to the detection values of the respective sensors 14 to the output units 15. Hereat, the user terminal 20 or the head region attaching device 10 outputs, based on an associative table between the detection values and the colors, the color associated with the detection value of each sensor 14 to the output unit 15 corresponding to each sensor 14. The user of the head region attaching device 10 and other equivalent persons are able to recognize the brain activity states through the output units 15 of the head region attaching device 10.

The output unit 15 capable of displaying plural items of information may also be mounted for one sensor 14. At this time, the variations in light quantity detected by the sensor 14, information about whether there are heartbeats extracted from the detection values of the brain activity states, variations in components of breathing, and other equivalent items, may be displayed on the output unit 15. The user and other equivalent persons of the head region attaching device 10 are able to acquire various items of information pertaining to the brain activity states through the output unit 15 of the head region attaching device 10. For example, the information of the heartbeats is normally displayed per sensor 14, thereby enabling confirmation such that the sensor 14 is attached in the good state. The normal display of the information of the heartbeats connotes, e.g., displaying variations on the order of 1 Hz as a frequency of the heartbeats. This is because the information of the heartbeats is not normally displayed when the sensor 14 is attached in an unpreferable state.

The configurations of the embodiment and the respective modified examples are implementable by being combined to the greatest possible degree.

### (Operations and Effects of Embodiment)

The information processing system irradiates the light from the light sources of the sensors 14 of the head region attaching device 10 attached to the user, and detects the light reflected by the head region. The information processing system determines the attaching state of the head region attaching device 10, based on the detected light (detection value) . The head region attaching device 10 displays the attaching state of each sensor 14 on the output unit 15. The user wearing the head region attaching device 10 or the assistant for the user recognizes the attaching state displayed on the output unit 15, and is thereby enabled to adjust the attaching state of the head region attaching device 10. The attaching state is displayed on the output unit 15 of the head region attaching device 10, thereby enabling the easy adjustment of the attaching state of the head region attaching device 10 and enabling a period of attaching time to be reduced.

### <Non-Transitory Computer Readable Recording Medium>

A program configured to cause a computer, other machines and apparatuses (which will hereinafter be referred to as the computer and other equivalent apparatuses) to attain any one of the functions, can be recorded on a non-transitory recording medium readable by the computer and other equivalent apparatuses . The computer and other equivalent apparatuses are made to read and execute the program on this non-transitory recording medium, whereby the function thereof can be provided.

Herein, the non-transitory recording medium readable by the computer and other equivalent apparatuses connotes a non-transitory recording medium capable of accumulating information instanced by data, programs and other equivalent information electrically, magnetically, optically, mechanically or by chemical action, which can be read from the computer and other equivalent apparatuses. Components instanced by the CPU and the memory configuring the computer are provided within such a non-transitory recording medium, in which the CPU may be made to run the program.

Among these non-transitory recording mediums, the mediums removable from the computer and other equivalent apparatuses are exemplified by a flexible disc, a magneto-optic disc, a CD-ROM, a CD-R/W, a DVD, a DAT, an 8 mm tape, and a memory card.

A hard disc, a Read-Only Memory (ROM) and other equivalent recording mediums are given as the non-transitory recording mediums fixed within the computer and other equivalent apparatuses.

### [Description of the Reference Numerals and Symbols]

- 10: head region attaching device
- 11: control unit
- 13: wireless communication unit
- 14: sensor
- 141: sensor
- 142: sensor
- 15: output unit
- 20: user terminal
- 21: CPU
- 22: memory
- 23: wireless communication unit
- 24: public network communication unit
- 25: display unit
- 26: operation unit
- 27: output unit
- 28: image capturing unit
- 29: positioning unit
- 2A: physical sensor unit

## Claims

1. A biophotonic measurement device comprising:
one or a plurality of photoirradiation means to irradiate rays of light;
one or a plurality of photodetection means to detect the rays of light of the photoirradiation means;
attaching means to attach the photo irradiation means and the photodetection means to a measurement examinee;
analyzing means to calculate an attaching state of the photoirradiation means and the photodetection means to the measurement examinee, based on a detection value detected by the photodetection means; and
output means, installed on the attaching means or on the photoirradiation means or on the photodetection means attached to the measurement examinee, to output the attaching state by use of the rays of light or sounds/voices.

2. The biophotonic measurement device according to claim 1, wherein the analyzing means determines whether there are hairs between the photoirradiation means or the photodetection means and the measurement examinee.

3. The biophotonic measurement device according to claim 1, further comprising a photoirradiator to ascertain the attaching state,
the analyzing means calculating the attaching state by using a quantity of light from the photoirradiator for a predetermined period of detection made by the photodetection means.

4. The biophotonic measurement device according to claim 1, wherein the attaching state is outputted to the output means of both of the photoirradiation means and the photodetection means.

5. The biophotonic measurement device according to claim 1, wherein the output means is enabled to output at least one of information about whether there are heartbeats, information about an increment and a decrement of the quantity of light and information about an increment and a decrement of breathing components.

6. A biophotonic measurement device comprising:
one or a plurality of photoirradiation means to irradiate rays of light;
one or a plurality of photodetection means to detect the rays of light of the photoirradiation means;
attaching means to attach the photo irradiation means and the photodetection means to a measurement examinee;
communication means to transmit a detection value detected by the photodetection means to an information processing apparatus, and to receive an attaching state of the photoirradiation means and the photodetection means to the measurement examinee from the information processing apparatus, the attaching state being calculated based on the detection value; and
output means, installed on the attaching means or on the photoirradiation means or on the photodetection means attached to the measurement examinee, to output the attaching state by use of the rays of light or sounds/voices.

7. An information processing program for a computer to execute:
calculating an attaching state of photo irradiation means and photodetection means to a measurement examinee, based on a detection value detected by the photodetection means of a biophotonic measurement apparatus including: one or a plurality of photoirradiation means to irradiate rays of light; one or a plurality of photodetection means to detect the rays of light of the photoirradiation means; attaching means to attach the photo irradiation means and the photodetection means to a measurement examinee; and output means, installed on the attaching means or on the photoirradiation means or on the photodetection means attached to the measurement examinee, to output the attaching state by use of the rays of light or sounds/voices; and
causing the output means to output the attaching state by use of rays of light or sounds/voices.

8. An information processing method by which a computer executes:
calculating an attaching state of photo irradiation means and photodetection means to a measurement examinee, based on a detection value detected by the photodetection means of a biophotonic measurement apparatus including: one or a plurality of photoirradiation means to irradiate rays of light; one or a plurality of photodetection means to detect the rays of light of the photoirradiation means; attaching means to attach the photo irradiation means and the photodetection means to a measurement examinee; and output means, installed on the attaching means or on the photoirradiation means or on the photodetection means attached to the measurement examinee, to output the attaching state by use of the rays of light or sounds/voices; and
causing the output means to output the attaching state by use of rays of light or sounds/voices.
